# EUROPEAN PATENT APPLICATION

(11) **EP 1 816 209 A2**
(43) Date of publication of application: **08.08.2007**
(21) Application number: 05799120.0
(22) Date of filing: 19.09.2005
(51) Int. Cl.: C12Q 1/04, C12N 1/20

(54) **CULTURE MEDIUM FOR THE DETECTION OF CLOSTRIDIUM PERFRINGENS**

(30) Priority: 17.09.2004 ES 200402323
(71) Applicant: UNIVERSIDADE DE SANTIAGO DE COMPOSTELA, 15782 Santiago de Compostela (ES)
(72) Inventor: ARAUJO PRADO, Manuel, 15782 Santiago de Compostela (ES)
(74) Representative: Carvajal y Urquijo, Isabel
(86) International application number: PCT/ES2005/000508
(87) International publication number: WO 2006/035092

(57) **Abstract**

The invention relates to a culture medium for detecting *Clostridium perfringens.* It comprises dissolving proteose peptone, microbiological peptone, yeast extract, sodium disulfite and ammonium ferric citrate in deionized water. After sterilization at 121°C/15 minutes and cooling, it is completed with the addition of cycloserine and 4-methylumbelliferyl phosphate disodium salt dissolved in deionized water and sterilized by filtration. The culture medium is distributed into 150 mL bottles with a nominal capacity of 50 mL for its storage in refrigeration and its use no more than 15 days after its preparation date. It is applicable in detecting *Clostridium perfringens* in water after the inoculation of 100 mL of sample in the bottles with 50 mL of medium and the incubation at 45°C for 18-20 hours in aerobic conditions.

## Description

**Field of the Art**: Microbiological water analysis

### State of the Art

This invention has as an object a culture medium and a process for detecting *Clostridium perfringens* in water using a presence-absence technique from 100 mL of sample.

*Clostridium perfringens* is an anaerobic, sporogenic gram positive bacillus which is probably the best choice as an indicator of the inactivation of viruses and oocysts in potable water, as well as the efficiency of disinfection treatments for water intended for human consumption (Payment, P. and Franco, E. 1993. Clostridium perfringens and somatic coliphages as indicators of the efficiency of drinking water treatment for viruses and protozoan cysts. Applied and Environmental Microbiology 59, 2418-2424). The spores of these Gram positive bacteria can provide a safety margin when purification treatment evaluation is involved. In this sense, Payment (Payment, P. 1999. Poor efficacy of residual chlorine disinfectant in drinking water to inactivate waterborne pathogens in distribution systems. Canadian Journal of Microbiology 45. 709-715) showed that sporulated bacteria such as *Clostridium perfringens* are barely affected by residual chlorine concentrations. Payment and Franco (*cited work*) proposed its use to replace the determination for viruses and parasites in water intended for human consumption. Several studies have further indicated that *Clostridium perfringens* can be a suitable indicator of the presence of pathogens of a fecal origin in surface water (Sorensen, D.L. et al. 1989. Clostridium perfringens as a point source indicator in non-point polluted streams. Water Research 23, 191-197;Payment, P. and Franco, E. 1993 (*cited work*))*.*

In this same line of thought, European Directive 98/83, in contrast with the previous Directive 80/778/EEC, has adopted as a criterion for controlling the sanitary quality of water intended for human consumption the determination for *Clostridium perfringens* (European Union (1998) Directive 98/83/EC of Council of 3 November 1998 on the quality of water intended for human consumption. Official Journal of the European Communities L330, 32-54). On the other hand, the mentioned Directive proposes the value of 0/100 mL as the microbiological standard for coliforms, *E. coli,* fecal enterococci and *Clostridium perfringens.* According to these standards, there is no doubt that a situation exists in which the number of indicator microorganisms in water is irrelevant because the mere presence of one of these microorganisms in a specific volume of water is enough to change its rating. On this basis it is logical to think that the most suitable analytical methodology for the determination of these microbiological parameters should be the one that is necessary for providing a response of the presence/absence in a previously defined volume of water.

All the commercially available culture mediums are designed to carry out the *Clostridium perfringens* count by means of the membrane filtration technique. Two groups can be distinguished among these culture mediums: traditional culture mediums and culture mediums based on the use of defined (chromogenic and fluorogenic) substrates.

Traditional culture mediums use a double strategy for the *Clostridium perfringens* count: reduction of sulfite by these bacteria or incorporation of one or several inhibitory agents (such as neomycin, polymyxin, sulfadiazine or cycloserine) of the microflora usually contained in the analyzed samples. All these mediums require cumbersome confirmation tests given that other species of the *Clostridium* genus can, generate a reaction similar to the reaction experienced by the colonies of *Cl. perfringens* in these mediums (Neut, C., et al. 1985. Rapid detection of Clostridium perfringens: comparison of lactose sulfite broth with tryptose-sulfite-cycloserine agar. Journal of the Association of Official Analytical Chemists 68, 881-883).

Commercially available culture mediums based on the use of defined substrates correspond to the chromogenic mCP medium and fluorogenic TSC agar with a selective supplement for recovering *Clostridium perfringens* (Merck). MCP agar is the culture medium established by European Directive 98/83 as a guideline for the *Clostridium perfringens* count in potable water samples. Several studies have demonstrated that in comparison with other commercially available culture mediums, this culture medium is not suitable for the *Clostridium perfringens* count from several types of water samples: water that is not very polluted, pre-treated water and underground water (Burger, J.S., Nupen, E.M. and Gravow, W.O.K. 1984. Evaluation of four growth media for the membrane filtration counting of Clostridium perfringens in water. Water SA 10, 185-188; Sartory, D.P. 1986. Membrane filtration enumeration of faecal clostridia and Clostridium perfringens in water. Water Research 10, 1255-1260; Sartory, D.P., Field, M., Curbishley, S.M. and Pritchard, A.M. 1998. Evaluation of two media for the membrane filtration enumeration of Clostridium perfringens from water. Letters in Applied Microbiology 27, 323-327; Araujo, M., Sueiro, R.A., Gómez, M.J. and Garrido, M.J. 2001. Evaluation of fluorogenic TSC agar for recovering Clostridium perfringens in groundwater samples. Water Science and Technology 43, 201-204; Araujo, M., Sueiro, R.A., Freire, B., Gómez, M.J., Garrido, M.J. 2004. Enumeration of Clostridium perfringens spores in groundwater samples: comparison of six culture media. Journal of Microbiological Methods, 57, 175-180).

The identification of *Clostridium perfringens* is possible by using a fluorogenic substrate (Manafi, M. 2000. New developments in chromogenic and fluorogenic culture media. International Journal of Food Microbiology 60, 205-218). Fluorogenic TSC agar (Merck) is a fluorogenic medium containing 4-methylumbelliferyl phosphate (MUP) disodium salt, a substrate for the enzyme acid phosphatase, which is a highly specific indicator for *Cl. perfringens* (Schallehn, G. and Brandis H. 1973. Phosphatase-reagent for quick identification of Clostridium perfringens. Zentralblatt fur Backteriologie und Hygiene, 1 Abteiling Originale A 225, 343-345; Eisgruber,H., Schalch, B., Sperner; B., and Stolle, A. 2000. Comparison of four routine methods for the confirmation of Clostridium perfringens in food. International Journal of Food Microbiology 57, 135-140). The confirmation of colonies with this culture medium is not necessary, being more suitable than the mCP medium for the detection of *Cl. perfringens* in underground water samples (Araujo, M., Sueiro, R.A., Gómez, M.J. and Garrido, M.J. 2001. Evaluation of fluorogenic TSC agar for recovering Clostridium perfringens in groundwater samples. Water Science and Technology 43, 201-204; Araujo, M., Sueiro, R.A., Freire, B., Gómez, M.J., Garrido, M.J. 2004. Enumeration of Clostridium perfringens spores in groundwater samples: comparison of six culture media. Journal of Microbiological Methods, 57, 175-180).

All the culture mediums, both traditional and chromogenic and fluorogenic, require the use of anaerobic conditions so that the characteristic colonies of *Cl. perfringens* can develop. This aspect along with the fact that with these mediums it is necessary to use the membrane filtration technique, makes the determination of *Clostridium perfringens* costly and complex, limiting the number of samples that can be simultaneously analyzed.

The presence-absence technique is a standard detection method for indicator microorganisms in water developed by Clark over 30 years ago (Clark, J.A. 1968. A presence-absence (PA) test providing sensitive and inexpensive detection of coliforms, fecal coliforms and fecal streptococci in municipal drinking water supplies. Canadian Journal of Microbiology 14, 13-18), consisting of the inoculation of a large portion of sample (100 mL) in a single culture bottle so as to obtain qualitative information on the presence or absence of microorganisms in water (APHA. 1998. Standard Methods for the examination of water and wastewater, 20th Edition. American Public Health Association. Washington D.C.).

In comparison with the standard methods for counting microorganisms in water, this technology is characterized by its simplicity and low cost, such that it allows analyzing a large number of samples in a short time period. The U.S. Environmental Protection Agency proposed the use of these analytical techniques to determine the presence of coliforms in drinking water over a decade ago (Federal Register (1988) National primary drinking water regulations: proposed water rule. Federal Register 53: 16348-16358).

According to the literature that was consulted, there is no process available for determining the presence-absence of *Cl. perfringens* in water by means of the technology of defined substrates. The present invention presents a fluorogenic culture medium based on acid phosphate activity for the detection of *Cl. perfringens* in water without the need of a later confirmation, using a presence-absence technique. Furthermore, the culture medium inoculated with the water sample does not require anaerobic incubation conditions.

### EXPLANATION OF THE INVENTION

The qualitative and quantitative composition of the new medium, which shall be referred to as PACp, is detailed in Table 1 in grams per liter. As can be seen, it contains a very rich nutritive base made up of proteose peptone no. 3, microbiological peptone and yeast extract, which provides the necessary nutrients for the growth of *Clostridium perfringens.* The culture medium further contains the following components which make it selective and differential for *Clostridium perfringens:*
- Sodium bisulfite and ammonium ferric citrate to emphasize the sulfite reduction capability.
- 4-methylumbelliferyl phosphate disodium salt, a fluorogenic compound forming the substrate for the acid phosphatase highly specific for *Clostridium perfringens.*

The culture medium with the indicated components allows selecting and differentiating the following types of cultures of members of the *Clostridium* genus:
- Cultures corresponding to *Clostridium* sulfite reducers, characterized by generating a black precipitate; this is due to the formation of ferrous sulfite due to sulfite reduction.
- Cultures corresponding to *Clostridium perfringens,* characterized by generating a black precipitate and emitting a bluish fluorescence when the medium is irradiated with a 366 nm lamp; this fluorescence is the result of the metabolization of 4-methylumbelliferyl phosphate by the acid phosphatase produced specifically by *Clostridium perfringens,* giving rise to the release of umbelliferone, a compound which emits bluish fluorescence when irradiated with a 366 nm lamp.

**Table 1. Components of the PACp medium.**

| **Components** | **Concentration (g/1000 mL)** |
|---|---|
| Proteose peptone no. 3 | 5.00 |
| Microbiological peptone | 15.00 |
| Yeast extract | 5.00 |
| D-cycloserine | 0.50 |
| Sodium disulfite | 1.00 |
| Ammonium ferric citrate | 0.25 |
| 4-methylumbelliferyl phosphate (MUP) disodium salt | 0.03 |

The preparation of the culture medium is carried out according to the following process:
1. Proteose peptone no. 3, the microbiological peptone and the yeast extract are weighted in a flask and dissolved in 980 mL of deionized water, heating if necessary,.
2. Then the preparation is completed with the addition of sodium disulfite and ammonium ferric citrate and heating until completely dissolved.
3. The resulting solution (solution A) is sterilized with moist heat at 121°C/15 minutes in an autoclave.
4. After sterilization it is left to cool at a temperature of less than 45°C.
5. The cycloserine and 4-methylumbelliferyl phosphate disodium salt are weighed in a flask and dissolved in 20 mL of deionized water (solution B). Once they are completely dissolved, they are sterilized by means of filtration.
6. The sterile solution B of cycloserine and 4-methylumbelliferyl phosphate disodium salt is added to sterile solution A and cooled at a temperature of less than 45°C. Then both solutions are mixed by means of stirring.
7. All the components are prepared at triple concentration and the culture medium resulting from the mixture of solutions A and B is distributed into 150 mL bottles with a nominal capacity of 50 mL in each.

The product must be stored in refrigeration until it is used, which should not occur any later than 15 days after its preparation.

The advantages involved in the use of the culture medium are summarized as:
- It allows detecting *Clostridium perfringens* in 18-20 hours, without needing subsequent confirmation, which means a reduction of time of over 24 hours.
- It allows detecting *Cl. perfringens* by incubating the culture medium in aerobic conditions, unlike what occurs with the other culture mediums, which require anaerobic conditions, which means reducing the complexity and the cost of the analysis.
- It allows detecting the presence of *Clostridium perfringens* in 100 mL of water by means of the presence-absence technique, which means the possibility of analyzing a larger number of samples in a shorter amount of time.

The culture medium was tested with pure cultures of different strains of *Clostridium perfringens* and of other species of the *Clostridium* genus. The results set forth in Table 2 indicate that the PACp medium is specific for detecting *Clostridium perfringens.* In the presence of different *Clostridium perfringens* strains, a positive reaction occurs in the inoculated PACp medium (black color and/or black precipitate and bluish fluorescence emission when irradiated with a 366 nm lamp), whereas in the presence of strains of other species of the *Clostridium* genus, the reaction in the inoculated PACp medium is negative (absence of black color and/or black precipitate and bluish fluorescence emission when irradiated with a 366 nm lamp).

**Table 2. Results obtained in the PACp medium inoculated with plant cells or spores of Clostridium genus strains from the Colección Española de Cultivos Tipo (CECT) (Spanish Culture Type Collection) and isolated from water samples.**

| | | | Aerobic conditions^{a} | | Anaerobic conditions | |
|---|---|---|---|---|---|---|
| Strains | Origin | No. | 1-10^{b} | >10 | 1-10 | >10 |
| *Cl. perfringens* | CECT | 5 | P^{c} | P | P | P |
| *Cl. perfringens* | Water | 50 | P | P | P | P |
| *Cl. acetobutylicum* | CECT | 2 | A | A | A | A |
| *Cl. bifermentans* | CECT | 1 | A | A | A | A |
| *Cl. butyricum* | CECT | 1 | A | A | A | A |
| *Cl. novyi* | CECT | 2 | A | A | A | A |
| *Cl. papyrosolvens* | CECT | 1 | A | A | A | A |
| *Cl. spiroforme* | CECT | 1 | A | A | A | A |
| *Cl. sporogenes* | CECT | 2 | A | A | A | A |

| | | | | | | |
|---|---|---|---|---|---|---|
| ^{a} Incubation conditions of the PACp medium. ^{b} Concentration of bacteria (colony forming units) inoculated in the PACp medium. ^{c}P, presence of a positive reaction for *Clostridium perfringens* (black color and/or precipitate and bluish fluorescence emission when irradiated with a 366 nm lamp) in the PACp medium; A, absence of a positive reaction in the PACp medium. | | | | | | |

On the other hand, the inoculation of the PACp medium with bacteria strains not belonging to the *Clostridium* genus resulted in a negative reaction in all cases, as can be seen in Table 3.

**Table 3. Results obtained in the PACp medium inoculated with microorganisms not belonging to the Clostridium genus and from the Colección Española de Cultivos Tipo (CECT) (Spanish Culture Type Collection).**

| Strains | Origin | No. | Aerobic conditions^{a} | Anaerobic conditions |
|---|---|---|---|---|
| *Lactobacillus* spp | CECT | 6 | A* | A |
| *Bacillus* spp | CECT | 5 | A | A |
| *Enterococcus* spp | CECT | 8 | A | A |
| *Staphylococcus* spp | CECT | 4 | A | A |
| *Enterobacteriaceae* | CECT | 15 | A | A |

| | | | | |
|---|---|---|---|---|
| *A, absence of a positive reaction in the PACp medium. | | | | |

### PROCESS FOR USING THE PACp MEDIUM

The PACp medium is used for detecting *Clostridium perfringens* from 100 mL of water using the following process based on the presence-absence technique:
a) Inoculating 100 mL of the water sample to be analyzed in 50 mL of the PACp medium at triple concentration contained in a container with a nominal capacity of 150 mL.
b) Incubating the inoculated PACp medium in an oven at 45.0°C ± 1.0°C for 18-20 hours in aerobic conditions.
c) Determining the presence of *Clostridium perfringens* according to the following reaction in the inoculated culture medium after incubation:
   - Development of a black color and/or black precipitate.
   - Bluish fluorescence emission when the culture medium with black color and/or black precipitate is irradiated with a 366 nm UV lamp.
d) Determining the absence of *Clostridium perfringens* according to the following reaction in the inoculated culture medium after incubation:
   - Absence of black color and/or black precipitate.
   - Absence of bluish fluorescence emission when the culture medium with black color and/or black precipitate is irradiated with a 366 nm UV lamp.

## Claims

1. A culture medium for detecting *Clostridium perfringens* **characterized by** consisting of 5.0 g/1000 mL proteose peptone, 15.0 g/1000 mL microbiological peptone, 5.0 g/1000 mL yeast extract, 1.0 g/1000 mL sodium disulfite, 0.25 g/1000 mL ammonium ferric citrate, 0.5g/ 1000 mL D-cycloserine, 0.03 g/1000 mL 4-methylumbelliferyl phosphate (MUP) disodium salt and deionized water at a volume of 1000 mL.

2. A process for preparing the culture medium according to claim 1, **characterized by** comprising the following steps:
a) Dissolving the proteose peptone no. 3, the microbiological peptone and the yeast extract in 980 mL of deionized water, heating if necessary,
b) Completing the preparation with the addition of sodium disulfite and ammonium ferric citrate and heating until completely dissolved,
c) Sterilizing the resulting solution (solution A) with moist heat at 121°C/15 minutes in an autoclave,
d) Leaving solution A to cool at a temperature of less than 45°C,
e) Dissolving the cycloserine and 4-methylumbelliferyl phosphate disodium salt in 20 mL of deionized water (solution B) and sterilizing by means of filtration,
f) Adding sterile solution B of cycloserine and 4-methylumbelliferyl phosphate disodium salt to sterile solution A and cooled at a temperature of less than 45°C and mixing both solutions by means of stirring,
g) Preparing all the components at triple concentration and distributing the culture medium resulting from the mixture of solutions A and B into sterile 150 mL bottles with a nominal capacity of 50 mL in each,
h) Storing the culture medium in refrigeration until it is used, no later than 15 days after its preparation,

3. A culture medium according to claim 1, **characterized by** its application for detecting *Cl. perfringens* in water by means of the presence-absence technique consisting of the inoculation of 100 mL of sample in bottles with 50 mL of medium and the incubation of the culture medium at 45°C ± 1.0°C for 18-20 hours in aerobic conditions.
